# EUROPEAN PATENT SPECIFICATION

(11) **EP 2 142 170 B1**
(45) Date of publication and mention of the grant of the patent: **16.11.2011**
(21) Application number: 08719894.1
(22) Date of filing: 03.04.2008
(51) Int. Cl.: A61K 9/00, A61K 9/20, A61K 47/42, A61K 47/30

(54) **A METHOD OF PRODUCING FAST DISSOLVING TABLETS**
VERFAHREN ZUR HERSTELLUNG VON SCHNELL AUFLÖSENDEN TABLETTEN
PROCÉDÉ DE PRODUCTION DE COMPRIMÉS FONDANT RAPIDEMENT

(30) Priority: 03.04.2007 EP 07394008; 09.04.2007 US 922313 P
(43) Date of publication of application: 13.01.2010
(73) Proprietor: Royal College of Surgeons in Ireland, Dublin 2 (IE)
(72) Inventor: RAMTOOLA, Zebunnissa, Dublin 9 (IE); PABARI, Ritesh, Dublin 2 (IE); KELLY, John, Dublin 9 (IE)
(74) Representative: Purdy, Hugh Barry
(86) International application number: PCT/IE2008/000036
(87) International publication number: WO 2008/120181

(56) References cited:
- WO-A-03/080023
- US-A- 5 178 878
- US-A1- 2003 215 502
- US-A1- 2007 082 048
- US-B1- 6 221 392

## Description

### Introduction

The invention relates to a method of producing fast dissolving tablets, and to fast dissolving tablets obtainable according to the method of the invention.

The use of conventional tablets is often challenging to geriatric, pediatric and uncooperative patients who have difficulties swallowing. Further, swallowing conventional tablets can be a problem when patients have a persistent cough or a gagreflex, or when water is unavailable. These problems have been partly addressed by the provision of fast dissolving tablets in recent years. These tablet forms are also known as FDDT (fast dissolving disintegration tablets), fast melt, or oral dissolving, tablets. Generally, these tablets include one or more hydrophilic disintegrants that, when placed on the tongue or in the oral cavity, rapidly absorb saliva and dissolve or disperse within less than one minute. A problem with the provision of these tablets is the need to provide a tablet that is sufficiently strong to withstand packaging, transport, and subsequent handling without breaking, yet capable of disintegrating rapidly when placed in the oral cavity. This problem has been addressed in a number of ways. Zydis (US Patents 4,305,502, 4,371,516 and 5738875) have addressed this issue by providing the tablet in the form of a friable freeze-dried matrix which, while dissolving rapidly in the mouth, is very fragile and requires the use of specialised packaging to ensure a minimum of handling of the tablet. Others have proposed the use of granulating, drying and tabletting technologies to provide a rapidly dispersible tablet that is more robust (EP0914818, WO2005/105049). While the tablets produced according to these process are less prone to breakage and, generally, do not require specialised packaging, the process involved in their manufacture is resource and energy intensive, and often requires the use of added functionality tablet excipients to enable processing of the materials.

US6221392 discloses the preparation of rapidly dissolvable tablets comprising (1) providing preferably 50-95% of sugar alcohols such as mannitol, dextrose, sorbitol, lactose, sucrose and 0-50%, preferably 2-5% of a disintegration agent such as cellulose and an active ingredient, (2) blending and (3) directly compressing at the compression force of 3-13 kN. This document discloses a long list of disintegrants among which superdisintegrants can be found.

US2003215502 relates to a method of producing of a fast melt dosage composition comprising (1) providing 42.5% mannitol and 15% of the superdisintegrant crospovidone and 40% active agent granules and lubricant, (2) blending and (3) directly compressing with a compression force from 3 to 5 kP.

US5178878 describes a preparation method for rapid disintegration tablets comprising (1) providing 41% release promoter mannitol and preferably 2-10% of disintegrant and 17.1 % active ingredient, (2) blending and (3) compressing with 1.5-4 kP. Among the useful disintegrants modified starches are mentioned which include superintegrants.

It is an object of the invention to overcome at least one of the above-referenced problems.

### Statements of Invention

The invention provides a method of producing a fast dissolving type tablet which disintegrates rapidly in the mouth, which has acceptable characteristics of hardness and friability which obviate the need for specialised packaging. The method employs simple processing technology, including direct compression tabletting, and employs a relatively simple blend of excipients which allows for ease of processing. Surprisingly, it has been found that tablets produced according to the method of the invention have a high crushing strength, low friability of below 1% as per USP method , and yet dissolve or melt rapidly in the mouth. Without being bound by theory, it is believed that the use of a fast dissolving sugar alcohol at a relatively high level, combined with the use of a super-disintegrant , allows tablets be formed by direct compression resulting in a robust tablet that is capable of rapidly disintegrating in the oral cavity. It has further been found that the use of flat faced toolings provides a tablet of better disintegration characteristics than those formed with bi-convex toolings. Further, it is has been found that the incorporation of the active component into microparticles or microcapsules, typically formed by spray drying or spray chilling, enables the use of direct compression in the formation of a tablet having acceptable dissolution and hardness characteristics.

According to the invention, there is provided a method of producing a fast dissolving type tablet comprising the steps of forming a mixture of components, the mixture comprising at least 50% of a fast dissolving sugar alcohol (w/w), 1 to 25% ofa superdisintegrant (w/w), and at least one active component, blending the mixture fora period of time, and directly compressing the blended mixture at a compression force of between 5 and 20 kN to form a fast dissolving type tablet in which the blended mixture is directly compressed using flat-faced toolings.

In a preferred embodiment, the process of the invention does not involve any granulation step, thereby making the process more energy efficient and cost effective. The process of the invention employs pre-processed (and commercially available) components, such as, for example, the fast dissolving sugar Mannitol 200, Mannitol 300, Ludipress, Sorbitol 300, however the process of the invention does not involve granulation. In one embodiment of the invention, the use of spray dried starches in the process is excluded.

Suitably, the fast dissolving sugar alcohol is selected from the group comprising: mannitol; sorbitol; erythritol; xylitol; lactose; dextrose; and sucrose. Preferably, the fast dissolving sugar alcohol is mannitol, ideally Mannitol 200. In one embodiment, the fast dissolving sugar alcohol comprises at least 50%, preferably at least 60%, and more preferably at least 70%, of the tablet (w/w). In one embodiment, the fast dissolving sugar alcohol comprises at least 80% or 85% of the tablet (w/w). In another embodiment, two different sugar alcohols are employed.

Typically, the super-disintegrant is selected from the group comprising: late nyl-2 crosscarmellose sodium, or combinations thereof. for example EXPLOTAB or a crospovidone such as KOLLIDON CL-SF. Typically, from 1 to 5% of the superdisintegrant is employed (w/w).

Typically, an optional osmotic agent is selected from the group comprising anhydrous organic acids and salts thereof. In one embodiment, the osmotic agent is anhydrous citric acid or sodium citrate. Suitably, the osmotic agent (or agents) comprise between 5 and 15%, preferably between 8 and 12%, and more preferably between 9% and 11%, of the tablet (w/w). Generally, either a disintegrant (or disintegrants) or an osmotic agent is employed.

In one preferred embodiment of the invention, the mixture of components additionally comprises a lubricant, typically selected from the group comprising: magnesium stearate; stearic acid, polyethylene glycol, polyoxyethylene- polyoxypropylene block copolymer (poloxamer). Suitably, the lubricant comprises between 0.1% and 5.0%, preferably between 0.2% and 1.0%, of the tablet (w/w).

In another embodiment, the lubricant, instead of or in addition to being included in the tablet formulation, is coated on to the faces of the tabletting dies.

Optionally, the mixture of components includes a flow enhancing agent such as, for example, talc or colloidal silicon dioxide, at from 0.1% to 3.0%, and preferably from 0.1% and 0.5%, of the tablet (w/w). The mixture of components optionally includes a flavouring agent (such as, for example, synthetic oils, natural oils, or extracts from plants or other suitable synthetic or naturally derived flavors), typically at a level ranging from 0.5 to 5 % of the tablet (w/w). The mixture of components may also include a surfactant or wetting agent (such as sodium lauryl sulphate, Tweens, Spans), typically at a level of from 0.1 to 3% of the tablet (w/w).

The method of the invention involves the tablets being formed in a direct compression process. Suitably, a tablet press is employed. The direct compression process employs substantially flat faced toolings, ideally with a bevelled edge. Thus, the thickness of the formed tablet will not vary considerably from the centre to the edges (unlike tablets produced using bi-convex toolings which are thicker in the middle that at the edges). Typically, the flat faced toolings have a uniform thickness, which will not vary in thickness between the centre and edge by more that +/- 5%, preferably 4%, preferably 3%, more preferably 2%, and ideally by more than 1%. In a particularly preferred embodiment, the tablet has diameter in the range of 5-20mm, preferably in the range of 10-15mm and more preferably 15mm. Typically, the tablet has a diameter of at least 10mm, at least 11mm, at least 12mm, at least 12mm, and at least 14mm. Preferably, the tablet has a thickness of between 1 and 4 mm, preferably between 1.5-3.5 mm.

In a preferred embodiment of the invention, the compression force employed in the direct compression process is from 8kN to 20kN, typically from 10kN to 15kN.

Suitably, one or more of the components is provided in the form of microparticles having an average diameter of less than 125µ. In a preferable embodiment of the method of the invention, at least one active component is provided in the form of microparticles.

Typically, the microparticles have an average diameter of less that 125µ, preferably less than 100µ, preferably less than 50µ, preferably less than 20µ, preferably less than 10µ, preferably less than 5µ, preferably less than 4µ, preferably less than 3µ, preferably less than 2p, preferably less than 1.5µ. In one embodiment, the microparticles have a mean diameter of about, or less than, 1.5µ. Generally, the microparticles are produced in a spray-drying or spray-chilling process.

In one embodiment, the microparticles have a solid or fluid core and a solid coating encapsulating the core (referred to hereafter as "microcapsules"). Such microcapsules may be formed in a process comprising the steps of providing a core-forming fluid stream and a coating-forming fluid stream, providing a two spray nozzle arrangement having a core nozzle disposed concentrically about a second nozzle, feeding the core-forming fluid stream to the core nozzle and the coat-forming fluid stream to the concentric nozzle to produce microcapsules, and solidifying the microcapsules immediately upon formation in a suitable gas.

Thus, the method of forming the microcapsules essentially comprises the steps of spraying a fluid stream through a nozzle to produce droplets, and drying (as in a spray drying process) or hardening (as in a spray chilling process) the droplets in air. Generally, the air will be hot air which dries the microcapsules as they leave the nozzle. However, in the case of spray chilling, in which case the fluid stream(s) comprise lipids and/or waxes and/or low melting point polymers, which are heated to melt these components, the microcapsules formed at the nozzle are solidified in cold air as opposed to hot air. General details of spray chilling methodology are available in the Quick Operation Guide to spray chilling, Buchi.

Generally, the method is characterised over conventional spray drying or spray chilling insofar as the nozzle comprises a core nozzle through which a core-forming fluid is sprayed and a second nozzle formed concentrically about the core nozzle and through which a coat-forming fluid stream is sprayed. The droplets formed by the double nozzle arrangement comprise a core of the first fluid and a coating of the second fluid.

In the case of spray drying, the hot gas is typically air or a different gas like an inert gas such as nitrogen, argon or other inert gases. In the case of spray chilling, air at ambient temperature of 45°C or below is generally used.

Typically, the core-forming fluid is a liquid or a gas. When it is of a liquid nature, it is selected from the group comprising: a solution; a suspension; a dispersion; a colloidal solution or dispersion; an oil; and an emulsion. Suitably, the core-forming liquid comprises an active compound or substance, optionally in combination with one or more pharmaceutically acceptable excipients. The active compound or substance may be any type of therapeutic, prophylactic, diagnostic, or prognostic agent. Further, it may be an agent used in imaging or labelling. In one preferred embodiment, the agent may be a pharmaceutically active agent that is required to be released in a controlled manner; thus, the coating may be designed to break down slowly in a physiological environment to release the encapsulated core over a period of time.

Typically, the core comprises a material/substance that is different to the material/substance of the coating.

Optionally the core may include a sustained release polymer with the coat being a second controlled release polymer with/without one or more targeting moieties.

In one embodiment, the core-forming fluid may comprise or consist of a gas or a volatile solvent such as but not limited to ethanol, acetone, ethylacetate. The gas may be selected from the group comprising: air; an inert gas; and a gas suitable for imaging applications. The use of a gaseous core finds particular application in microcapsules for pulmonary delivery, the gaseous core providing a microcapsule of low density more suited for delivery as an aerosol.

In one embodiment of the invention, the coat-forming fluid comprises a coating material capable of forming a film or wall around the core material. Suitably, the coat forming fluid comprises a component selected from the group comprising: polymer; lipid; wax; surfactants; surface stabilising agents; and ligands suitable for targeting the microcapsules to a specific desired site of action in the body. Suitably, the polymer is selected from the group comprising: methacrylate polymers such as Eudragit polymers; ethylcellulose polymers; biodegradable polyesters such as poly-lactide (PLA),poly-glycolide (PGA),_and copolymers of lactic and glycolic acid, poly-lactide-co-glycolide (PLGA, poly-caprolactone (PCA); poly-amino acids; albumin; gelatine; alginate; and chitosan. Other suitable film-forming or wall-forming materials will be known to those skilled in the art.

The coat-forming fluid preferably comprises one or more agents selected from the group comprising: a pharmaceutically active agent; a taste masking agent (i.e. a sweetener); an agent that is liable to dissolution, swelling or degradation under certain defined (possibly physiological) conditions (a pH sensitive polymer, starch and starch derivatives, etc); a targeting compound (a ligand to a cell surface receptor overexpressed in tumour cells, i.e. vacuolar ATPases); an enhancer (short and medium chain fatty acids and their salts); a surfactant or wetting agent (tween, poloxamer, etc); and a surface stabilising agent (poloxamer, polyvinylpyrrolidone, etc).

In another embodiment, the coating may comprise a targeting moiety which is designed to target cells, tissues or organs to deliver the active agent. For example, the targeting moiety could be a ligand having a high affinity for a receptor that is highly expressed on the surface of tumour cells, i.e. ligands to vacuolar proton ATPases.

When spray chilling is employed, the coat-forming fluid may comprise lipids including phospholipids, waxes, surfactants or low melting point polymers which have a melting point of up to 75°C.

In one embodiment, the core nozzle has a diameter of between 0.7 and 2 mm. Typically, the concentric nozzle has a diameter of between 1.4 and 4 mm. Preferably, the core nozzle has a diameter of about I mm and the concentric nozzle has a diameter of about 2mm. Alternatively, the core nozzle has a diameter of about 1.5mm and the concentric nozzle has a diameter of about 3mm. Alternatively, the core nozzle has a diameter of about 2mm and the concentric nozzle has a diameter of about 4mm. Generally, the diameter of the core nozzle is between 40% and 60%, preferably about 50%, the diameter of the concentric nozzle.

Suitably, the core and coat-forming fluid streams have a flow rate of up to 25ml/min depending on the viscosity of the solution and the pump setting.

The droplets formed by the nozzle are dried as they leave the nozzle and pass through the heated gas. As it is a spray drying process, the gas is hot air or a heated inert gas such as nitrogen, typically having an inlet temperature of between 80°C and 220°C (preferably between 90°C and 110°C, and ideally about 100°, when heated nitrogen is used). Suitably, the heated nitrogen has an outlet temperature of between 40°C and 70°C.

When heated air is used, the inlet temperature has a range 120-220°C and the outlet temperature between 60°C and 160°C.

The methods described above are suitable for forming microcapsules having a core encapsulated by a single coat. However, the method may be employed to produce microcapsules having two or more coats. Thus, the nozzle may comprise at least one further nozzle formed concentrically about the second nozzle and through which a further coat-forming fluid stream is sprayed. The use of multiple coats can have advantages in the sequential and controlled delivery of more than one active agent. Thus, for example, a microcapsule may be formed comprising a core containing a first active, a first coat comprising a second active, and an outer coat. In use, such a microcapsule would have a delayed release of the actives, with the second active being released first (but only after the outer coat is degraded), and the first active being delivered last. Alternatively, the components of the microcapsule could be chosen such that a sustained release of active is achieved through the provision of a number of different coats.

In a preferred embodiment of the method of the invention, the active is a highly potent pharmaceutical comprising less that 5%, preferably less that 4%, preferably less that 3%, preferably less that 2%, preferably less than 1%, preferably less than 0.5%, and preferably less than 0.2%, of the tablet (w/w). In such circumstances, the active may be provided is the form of microparticles, or microcapsules, having a average diameter of less than 125µ, preferably less than 100p, preferably less than 50µ, preferably less than 40µ, preferably less than 30µ, preferably less than 20µ, preferably less than 10µ, preferably less than 5µ, preferably less than 4µ, preferably less than 3µ, preferably less than 2µ, and preferably less than 1.5µ. The provision of the active in the form of a microparticle or microcapsule ensures a homogenous distribution of small particles of the active in the fast dissolving tablet, thereby increasing the bioavailability. Thus, the invention also relates to a method of producing a tablet of the type comprising a highly potent pharmaceutical active present in the tablet at less than 5%, preferably less that 4%, preferably less that 3%, preferably less that 2%, and preferably less than 1%, of the tablet (w/w), the method comprising the steps of producing a microcapsule or microparticle containing the highly potent active, blending the formed microparticles or microcapsules with other tablet excipients, and forming the tablet using suitable means. Typically, the tablet is formed by direct compression, ideally using flat-faced toolings, however other tabletting means are also envisaged. Examples of such highly potent pharmaceutical actives include steroids and peptide therapeutics such as desmopressin. Other examples of highly potent actives that are used in small quantities will be well known to those skilled in the art.

The invention also relates to a directly compressed fast dissolving tablet comprising at least one fast dissolving sugar alcohol, at least one super-disintegrant, and at least one an active component, and optionally a lubricant.

The invention also relates to a fast dissolving tablet consisting essentially of a fast dissolving sugar alcohol, for example mannitol, preferably mannitol 200, and a superdisintegrant such as EXPLOTAB. The tablet is substantially flat-faced. Typically, a ratio of the thickness of the tablet at its centre and its edge is not greater than 105:100, preferably not greater than 104:100, preferably not greater than 103:100, preferably not greater than 102:100, and ideally not greater than 101:100.

The invention also relates to a directly compressed fast dissolving tablet consisting essentially of:
- 50% to 80% of a fast dissolving sugar alcohol;
- 2% to 10% superdisintegrant (w/w)
- 0.1% to 25% of active component (w/w);
- 0% to 1% of lubricant (w/w) and
- optionally, one or more of flavouring agents, flow enhancers or permeability enhancers,
the tablet having a disintegration time of less than 60 seconds and a hardness of greater than 40 Newtons. The tablet is substantially flat-faced. EXPLOTAB is typically employed as superdisintegrant.

The invention also relates to a directly compressed fast dissolving tablet consisting essentially of a fast dissolving sugar alcohol, a superdisintegrant, and active agent, and, optionally, one or more of flavoring agents, flow enhancers or permeability enhancers. The tablet has a disintegration time of less than 60 seconds and a hardness of greater than 40 Newtons. The tablet is substantially flat-faced.

Ideally, the tablet is circular or oval and typically has a diameter of between 5 and 20mm, and preferably a thickness of between 1 and 5mm.

Generally, the fast dissolving sugar alcohol is selected from the group comprising: mannitol; sorbitol; erythritol; xylitol; lactose; dextrose; and sucrose. Preferably, the fast dissolving sugar alcohol is mannitol, ideally Mannitol 200. In one embodiment, the fast dissolving sugar alcohol comprises at least 50%, preferably at least 60%, and more preferably at least 70%, of the tablet (w/w). In one embodiment, the fast dissolving sugar alcohol comprises at least 80% of the tablet (w/w). In another embodiment, two different sugar alcohols are employed.

Typically, the optional osmotic agent is selected from the group comprising anhydrous organic acids and salts thereof. In one embodiment, the osmotic agent is anhydrous citric acid or sodium citrate. Suitably, the osmotic agent (or agents) comprise between 5 and 15%, preferably between 8 and 12%, and more preferably between 9% and 11%, of the tablet (w/w).

In one preferred embodiment of the invention, the mixture of components additionally comprises a lubricant, typically selected from the group comprising: magnesium stearate; stearic acid, polyethylene glycol, polyoxyethylene- polyoxypropylene block copolymer (poloxamers). Suitably, the lubricant comprises between 0.1% and 5.0%, preferably between 0.2% and 1.0%, of the tablet (w/w). In another embodiment, the lubricant, instead of or in addition to being included in the tablet formulation, is coated on to the faces of the tabletting dies.

Optionally, the mixture of components includes a flow enhancing agent such as, for example, talc or colloidal silicon dioxide, at from 0.1% to 3.0%, and preferably from 0.1% and 0.5%, of the tablet (w/w). The mixture of components optionally includes one or a mixture of flavouring agent (such as, for example, synthetic oils, natural oils, or extracts from plants, other synthetic or natural flavors), typically at a level ranging from 0.5 to 5 % of the tablet (w/w). Preferably the flavouring agent included is a combination of flavours so as to enhance the taste masking of active ingredients. Examples of mixtures flavours include raspberry and mint, chocolate and mint, chocolate and vanilla, strawberry and vanilla, mixture of citrus flavours such as lemon and orange. The mixture of components may also include a surfactant or wetting agent (such as sodium lauryl sulphate, Tweens, Spans), typically at a level of from 0.1 to 3% of the tablet (w/w).

In one embodiment, the mixture of components includes a permeability enhancer selected from the group consisting of bile salts such as sodium glycocholate; chitosan derivatives; or salts and derivatives of short and medium chain fatty acids (C6-C12) such as sodium caprate, which are designed to enhance the buccal and/ oral permeability and absorption of poorly permeable actives.

In another embodiment, the mixture of component includes a surfactant or wetting agent such as for example, Sodium lauryl sulphate or poloxamer designed to enhance the solubility and absorption of poorly soluble actives

The tablet is substantially flat-faced and preferably a bevelled edge. Typically, the tablet has a diameter of at least 5mm, preferably at least 10mm, preferably at least 12mm, preferably at least 13mm, preferably at least 14mm, and preferably at least 15mm. Ideally, the tablet has a thickness of from 1 to 4mm, preferably from 1.5 to 2.5mm. In one preferred embodiment of the invention, at least one of the components of the tablet is provided in the form of microparticles or microcapsules having an average dimension of 125µ or less. Typically, the active is provided in the form of microparticles or microcapsules having an average dimension of 125µ or less.

The invention also relates to a directly compressed fast dissolving tablets obtainable by the process of the invention.

The tablets of, and obtainable according to the process of, the invention suitably have a disintegration time of less than 60s, 50s, 45s, 40s, 35s, 30s, 25s, 20s, 15s, or 10s.

The tablets of, and obtainable according to the process of, the invention suitably have a friability of less than 1%, and most preferably less than 0.5% as determined using the USP method

The tablets of, and obtainable according to the process of, the invention suitably have a weight variation of less than 5%, preferably less than 3%, preferably less than 2%, and most preferably less than 1%.

Preferably, the tablet of, and obtainable according to the process of, the invention have a hardness of greater than 30 Newtons, preferably greater than 35 Newtons, preferably greater than 40 Newtons, preferably greater than 45 Newtons, preferably greater than 50 Newtons, preferably greater than 55 newtons, preferably greater than 60 Newtons, and preferably greater than 65 Newtons.

Typically, the tablet of, and obtainable according to the process of, the invention have a friability of 0 to less than 1% w/w according to USP method

The invention will be more clearly understood from the following description of some embodiments thereof, given by way of example only.

### Detailed Description of the Invention

The examples below provide a number of fast dissolving tablets formed according to the process of the invention. The characteristics of the tablets were determined as follows Disintegration time (PharmaTest Disintegration tester PTZ Auto, PTFE Germany) Hardness or Crushing strength (PharmaTest tablet hardness tester, PTB 41 E, Germany) Uniformity of weight (Sartorius, Model: CP225D)

Thickness (Digital caliper, Workzone UK)

Friability Tester (PharmaTest, PTFE Germany)

### Example 1

The following tabletting excipients were weighed and blended for 5 minutes in a sealed plastic bag. 37.25g of Mannitol 200, 5g of Explotab, 5 g of calcium silicate and 2.5 g of sodium diclofenac uncoated. After blending for 5 minutes, 0.25g of magnesium stearate was added and blended gently x 1 minute. The powder blend was then transferred to the hopper of a Piccola tablet press (An 8 station Rotary Tablet Press operating at a speed of 14 tablets per minute) fitted with 15 mm flat faced, bevelled edge round toolings and compressed at a force of 15 kN. Tablets were produced at a target tablet weight was 500mg. Tablets obtained were tested for weight uniformity, hardness and disintegration times. Tablets showed an average weight of 517mg, a hardness of 54 Newtons and a disintegration time of 1 minute and 20 seconds.

### Example 2

Example 1 was repeated using Eudragit E coated sodium diclofenac prepared by spray drying a solution of sodium diclofenac and Eudragit E in ethylacetate (as described below in Example 8). The formula used was adjusted to keep the content of diclofenac at 25mg/500mg tablet weight. 10g of Eudragit E coated sodium diclofenac was used instead of 2.5g of sodium diclofenac and was blended with 29.75g of Mannitol 200, 5g of Explotab and 5 g of calcium silicate. After 5 minute blending, 0.25g of magnesium stearate was added and blended gently x 1 minute. Tablets were produced at a compression force of 12 kN and showed a hardness of 72 Newtons and a disintegration time of 40 seconds. Average tablet weight was 420mg.

### Comparative Example 3

Placebo FDDTs were manufactured using a blend containing 44.75g of Mannitol 200, 5g of anhydrous citric acid and 0.25g of magnesium stearate. The blend was prepared as in Example 1 and tablets were produced at a compression force of 10 KN. Tablets produced had an average weight of 520mg and showed a hardness of 56 Newtons and a disintegration time of 16 seconds.

### Comparative Example 4

Example 3 was repeated using sodium citrate instead of anhydrous citric acid. The tablets produced had an average weight of 512mg and showed a hardness of 46 Newtons and a disintegration time of 9 seconds.

### Comparative Example 5

The following tabletting excipients were weighed and blended for 5 minutes in a sealed plastic bag. 39.75g of Mannitol 200 and 5g of SSG. After blending for 5 minutes, 0.25g of magnesium stearate was added and blended gently x I minute. The powder blend was then transferred to the hopper of a Piccola tablet press fitted with 15 mm flat faced, bevelled edge round tool ings and compressed at a force of 10 kN and speed of 14 tablets per minute. Tablets were produced at a target tablet weight was 500mg. Tablets obtained were tested for weight uniformity, hardness and disintegration times. Tablets showed an average weight of 551mg, a hardness of 37 Newtons and a disintegration time of 37 seconds.

### Comparative Example 6

Example 5 was repeated, but employing a compression force of 15 kN. Tablet were produced at a target tablet weight was 500mg. Tablets obtained were tested for weight uniformity, hardness and disintegration times. Tablets showed an average weight of 546mg, a hardness of 54 Newtons and a disintegration time of 37 seconds.

### Comparative Example 7

Example 5 was repeated, but employing a compression force of 20 kN. Tablet were produced at a target tablet weight was 500mg. Tablets obtained were tested for weight uniformity, hardness and disintegration times. Tablets showed an average weight of 541 mg, a hardness of 97 Newtons and a disintegration time of 42 seconds.

### Example 8

A solution of sodium diclofenac and Ethylcellulose was prepared by dissolving 5.0 g of sodium diclofenac and 15.0 g of Ethylcellulose polymer in 200mls of ethanol using a magnetic stirrer. The solution was spray dried using the Bucchi 290 Laboratory spray drier to form microparticles. This was repeated twice and the microparticles from the 3 batches were blended. The average diameter of the blended microparticles was 8.42 ± 0.68 microns and the sodium diclofenac loading was at 24:80 (w/w). The sodium diclofenac microparticles were blended with mannitol, Kollidon CL-SF and chocolate flavouring at the following weight ratios of 20g of sodium diclofenac microparticles: 70.5g of Mannitol 200: 5 g Kollidon CL-SF: 4g Chocolate flavouring. 0.5 g of Magnesium stearate was then added to the blend. This blend was then tabletted using 15mm flat beveled edge tablet toolings at a compression force of 10kN and a speed of 14 tablets per minute. Tablets obtained had a weight uniformity of 515.92 ± 15.51 mg, a hardness of 39.01 ± 5.17 Newtons, a disintegration time of 32 ± 3 seconds, a friability of 0.58% and a sodium diclofenac content of 27.00 ± 1.22 mg.

### Example 9

The following tabletting excipients were weighed and blended for 5 minutes in a sealed plastic bag. 93.9g of Mannitol 200, and 5g of Kollidon CL-SF and 0.6 g of raspberry flavouring. After blending for 5 minutes, 0.5g of magnesium stearate was added and blended gently x 1 minute. The powder blend was then transferred to the hopper of a Piccola tablet press fitted with 15 mm flat faced, bevelled edge round toolings and compressed at a force of 15 kN and speed of 14 tablets per minute. Tablets were produced at a target tablet weight was 500mg. Tablets obtained were tested for weight uniformity, hardness, friability and disintegration times. Tablets showed an average weight of 499 ± 15.51 mg, a hardness of 44.58 ± 2.98 Newtons and a disintegration time of 22 ± 2 seconds and a friability of 0.89%.

### Example 10

The following tabletting excipients were weighed and blended for 5 minutes in a sealed plastic bag. 91.7g of Mannitol 200, and 5g of Kollidon CL-SF, 2g of chocolate and 0.8 g of mint flavouring. After blending for 5 minutes, 0.5g of magnesium stearate was added and blended gently x I minute. The powder blend was then transferred to the hopper of a Piccola tablet press fitted with 10mm flat faced, bevelled edge round toolings and compressed at a force of 10 kN. Tablets were produced at a speed of 98 tablets per minute and at a target tablet weight of 200mg. Tablets obtained were tested for weight uniformity, hardness, friability and disintegration times. Tablets showed an average weight of 202 ± 0.00 mg, a hardness of 44.8 ± 1.35 Newtons and a disintegration time of 20.3 ± 4.93 seconds and a friability of 0.00%.

### Example 11

Example 10 was repeated at a higher tabletting speed of 196 tablets per minute. Tablets obtained were tested for weight uniformity, hardness, friability and disintegration times. Tablets showed an average weight of 197.16 ± 2.41 mg, a hardness of 38 ± 0.85 Newtons and a disintegration time of 28.3 ± 5.03 seconds and a friability of 0.09 %.

### Example 12

Example 10 was repeated using 13 mm flat faced, bevelled edge round toolings a compression force of 12 kN, a speed of 14 tablets per minute and a tablet target weight of 300mg. Tablets obtained were tested for weight uniformity, hardness, friability and disintegration times. Tablets showed an average weight of 297.52 ± 1.66 mg, a hardness of 30.30 ± 2.34 Newtons and a disintegration time of 18.20 ± 2.15 seconds and a friability of 0.00 %.

### Example 13

Example 12 was repeated using a formulation blend of 92.9g of Mannitol 200, and 5g of Kollidon CL-SF, 0.8 g of raspberry and 0.8 g of mint flavouring. After blending for 5 minutes, 0.5g of magnesium stearate was added and blended gently x 1 minute. Tablets obtained were tested for weight uniformity, hardness, friability and disintegration times. Tablets showed an average weight of 302.16 ± 2.40 mg, a hardness of 31.42 ± 1.59 Newtons and a disintegration time of 16.4 ± 1.78 seconds and a friability of 0.00 %.

### Example 14

Example 13 was repeated using spray dried Mannitol (Mannogem EZ) instead of Mannitol 200. Tablets obtained were tested for weight uniformity, hardness, friability and disintegration times. Tablets showed an average weight of 304.83 ± 5.03 mg, a hardness of 15.37 ± 4.13 Newtons and a disintegration time of 6.9 ± 1.6 seconds and a friability of 100% (all tablets broken).

### Example 15

The following tabletting excipients were weighed and blended for 5 minutes in a sealed plastic bag 81.7. g of Mannitol 200, 10g simvastatin and 5g of Kollidon CL-SF, 2g of chocolate and 0.8 g of mint flavouring. After blending for 5 minutes, 0.5g of magnesium stearate was added and blended gently x I minute. The powder blend was then transferred to the hopper of a Piccola tablet press fitted with 13mm flat faced, bevelled edge round toolings and compressed at a force of 12 kN. Tablets were produced at a speed of 14 tablets per minute and at a target tablet weight of 300mg. Tablets obtained were tested for weight uniformity, hardness, friability and disintegration times. Tablets showed an average weight of 308.07 ± 2.47 mg, a hardness of 26.08±Newtons and a disintegration time of 24.67 ± 2.52 seconds and a friability of 0.00%. The simvastatin content of the tablets assayed by HPLC analysis was 28.10 ± 1.99 mg/tablet

### Comparative Example 16

The following tabletting excipients were weighed and blended for 5 minutes in a sealed plastic bag 79.5g of Mannitol 200, and 10g of calcium silicate and 10 g of SSG. After blending for 5 minutes, 0.5g of magnesium stearate was added and blended gently x 1 minute. The powder blend was then transferred to the hopper of a Piccola tablet press fitted with 10mm flat faced, bevelled edge round toolings and compressed at a force of 10 kN. Tablets were produced at a speed of 14 tablets per minute and at a target tablet weight of 300mg. Tablets obtained were tested for weight uniformity, hardness, friability and disintegration times. Tablets showed an average weight of 300.37 ± 1.92mg, a hardness of 45.35 ± 3.84 Newtons and a disintegration time of 51.2 ± 3.33 seconds and a friability of 0.17%.

### Comparative Example 17

Example 16 was repeated using 10mm round concave toolings to produce biconvex tablets. Tablets obtained were tested for weight uniformity, hardness, friability and disintegration times. Tablets showed an average weight of 295.17 ± 3.38 mg, a hardness of 84.19 ± 3.38 Newtons and a disintegration time of 105.9 ± 3.75 seconds and a friability of 0.00%.

### Comparative Example 18

Example 16 was repeated twice using 13 mm flat faced beveled edge round toolings and 13mm round concave toolings. Tablets obtained were tested for weight uniformity, hardness, friability and disintegration times. The 13 mm flat faced, beveled edge tablets showed an average weight of 490.95 ± 2.37 mg, a hardness of 30.29 ± 1.02 Newtons and a disintegration time of 37.9 ± 2.81 seconds and a friability of 0.36%.

The 13 mm biconvex tablets showed an average weight of 493.5 ± 5.03 mg, a hardness of 31.64 ± 1.94 Newtons and a disintegration time of 105.1 ± 11.50 seconds and a friability of 0.00%.

### Example 19

The following tabletting excipients were weighed and blended for 5 minutes in a sealed plastic bag 93.4 g of Mannitol 200, 5g of Kollidon CL-SF and 0.6g of raspberry flavouring and 0.5g of Novamint fresh peppermint. After blending for 5 minutes, 0.5g of magnesium stearate was added and blended gently x I minute. The powder blend was then transferred to the hopper of a Piccola tablet press fitted with 20mm flat faced, bevelled edge round toolings and compressed at a force of 20 kN. Tablets were produced at a speed of 14 tablets per minute and at a target tablet weight of 1000mg. Tablets obtained were tested for weight uniformity, hardness, friability and disintegration times. Tablets showed an average weight of 1009.98 ± 10.92mg, a hardness of 41.10 ± 1.70 Newtons and a disintegration time of 23.9 ± 2.51 seconds and a friability of 0.40%.

### Example 20

The following tabletting excipients were weighed and blended for 5 minutes in a sealed plastic bag 135.75 g of Mannitol 200, 7.5g of Kollidon CL-SF and 6g of chocolate flavouring. After blending for 5 minutes, 0.75g of magnesium stearate was added and blended gently x I minute. The powder blend was then transferred to the hopper of a Piccola tablet press fitted with 15mm flat faced, bevelled edge round toolings and compressed at a force of 20 kN. Tablets were produced at a speed of 14 tablets per minute and at a target tablet weight of 500mg. Tablets obtained were tested for weight uniformity, hardness, friability and disintegration times. Tablets showed an average weight of 497.57 ± 2.91 mg, a hardness of 50.39 ± 3.02 Newtons and a disintegration time of 25.0 ± 3.0 seconds and a friability of 0.40%.

### Stability testing of tablets

Tablets prepared in example 20 were placed in an amber glass tablet container and the container was stored at ambient conditions in a non controlled laboratory environment. At suitable time intervals of 1, 6, 9 and 12 months, samples were removed and tested for weight uniformity, hardness, friability and disintegration times. The data shown in table below shows minimal change in hardness, disintegration time and friability of the tablets over the storage period of 12 months.

| **Time (Months)** | **Weight Variation (mg)** | **Hardness (Newton)** | **Disintegration Time (seconds)** | **Friability (% wt loss) [n = 10]** |
|---|---|---|---|---|
| 0 | 497.57 ± 2.91 | 50.39 ± 3.02 | 25 ± 3 | 0.4 |
| 6 | 502.30 ± 10.40 | 49.38 ± 3.17 | 19 ± 1.41 | 0.4 |
| 9 | 496.21 ± 2.79 | 47.92 ± 2.70 | 23.83 ± 2.70 | 0 |
| 12 | 496.10 ± 4.53 | 49.43 ± 2.11 | 22.17 ± 5.64 | 0 |

As used herein the term "fast dissolving sugar alcohol" is meant to describe those sugar alcohols that dissolve quickly in the salivary conditions of the oral cavity. To determine the dissolution rate of sugar alcohol the following method is used, which simulates the environment of the oral cavity:
1) 2.5 grams of sugar alcohol material is weighed and hand pressed into a tablet. The tablet is pressed to a desired tablet "crush" hardness of approximately 8000grams. The tablet "crush" hardness is measured by calculating the force, in grams, needed to crush the tablet.
2) To determine the tablet dissolution in the salivary environment of the oral cavity, commercially available artificial saliva, such as sterile refined porcine gastric mucin, is used. Saliva Orthana, manufactured by A/S Orthana Keisk Fabrik, Kastrup, Denmark is a suitable artificial saliva.
3) In a beaker, 450 mL (milliliters) of the artificial saliva is heated to 32°C and stirred at 300 rpm (revolutions per minute) with a magnetic stiner. 40 mL of the preheated saliva is removed and placed in a 60mL beaker and stirred at 400 rpm.
4) The sugar alcohol tablet is added to the artificial saliva. The time in seconds for the tablet to breakup from a tablet shape into pieces is recorded as the tablet breakdown time. The time in seconds that the tablet takes to dissolve completely into the solution is recorded as the dissolution time.

Fast dissolving sugar alcohols are those sugar alcohols typically with a dissolution time of about 200 seconds or less based on the above method, in one embodiment about 150 seconds or less.

In this specification, the term "fast dissolving type tablets" should also be understood to include chewable tablets. Further, the tablets of, and obtainable by the process of, the invention find utility for both human and animal use, and for delivery of pharmaceutical, dietary, nutraceutical, and other forms of active components. Further, they may be provided in the form of tablets intended to be dissolved in a solution prior to ingestion, and also oral, vaginal and other routes of administration. The tablets of, and obtainable by the process of, the invention are also useful for the delivery of macromolecules, unpalatable actives, highly potent actives, and actives that are subject to first-pass metabolism, both by means of local and systemic administration. They are also useful for the sub-lingual delivery of actives.

## Claims

1. A method of producing a fast dissolving type tablet comprising the steps of forming a mixture of components, the mixture comprising at least 50% of a fast dissolving sugar alcohol (w/w), 1 to 25% of a superdisintegrant (w/w), and at least one active component, blending the mixture for a period of time, and directly compressing the blended mixture at a compression force of between 5 and 20kN to form a fast dissolving type tablet, in which the blended mixture is directly compressed using flat-faced toolings.

2. A method as claimed in Claim 1 in which the blended mixture is directly compressed to form a fast dissolving type tablet having a thickness of between 1 and 5mm, and/or the blended mixture is directly compressed to form a fast dissolving type tablet having a diameter of between 5 and 20mm.

3. A method as claimed in any preceding Claim in which the fast dissolving sugar alcohol is selected from the group comprising: mannitol; sorbitol; erythritol; xylitol; lactose; dextrose; and sucrose, and/or the fast dissolving sugar alcohol comprises at least 60% or 80% of the tablet (w/w), and/or the tablets are directly compressed to form a fast dissolving type tablet with a bevelled edge, and/or one or more of the components is provided in the form of microparticles having an average diameter of less than 50µ.

4. A method as claimed in any preceding Claim in which the active is a highly potent pharmaceutical comprising less that 5% of the tablet (w/w), and in which the highly potent pharmaceutical active is optionally selected from the group consisting of: steroids; hormones; peptide therapeutics; and protein therapeutics.

5. A fast dissolving tablet obtainable by the method of any of Claims 1 to 4.

6. A directly compressed fast dissolving tablet comprising at least 50% of a fast dissolving sugar alcohol (w/w), 1 to 25% of a superdisintegrant (w/w), at least one active component, and optionally 0.1 to 5% of a lubricant (w/w), wherein the tablet has a hardness of greater than 30 Newtons, a friability of less than 1%, and a disintegration time of less than 60 seconds, and in which the tablet is flat-faced.

7. A directly compressed fast dissolving tablet as claimed in Claim 6 and consisting essentially of:
- 50% to 80% of a fast dissolving sugar alcohol (w/w);
- 2% to 10% superdisintegrant (w/w);
- 0.1 % to 25% of active component (w/w);
- 0% to 1% of lubricant (w/w), and
- optionally, one or more of flavouring agents, flow enhancers or permeability enhancers,
the tablet having a hardness of greater than 40 Newtons.

8. A directly compressed fast dissolving tablet as claimed in any of Claims 5 to 7 in which the tablet has a diameter of between 5 and 20mm and, optionally, a thickness of between 1 and 5mm.

9. A directly compressed fast dissolving tablet as claimed in any of Claims 5 to 8 in which the fast dissolving sugar alcohol is selected from the group comprising: mannitol; sorbitol; erythritol; xylitol; lactose; dextrose; and sucrose, and/or the fast dissolving sugar alcohol comprises at least 60% or 80% of the tablet (w/w), and/or the tablet has a bevelled edge.

10. A directly compressed fast dissolving tablet as claimed in any of Claims 5 to 9 in which one or more of the components is provided in the form of microparticles having an average diameter of less than 50µ.

11. A directly compressed fast dissolving tablet as claimed in any of Claims 5 to 10 in which the active is a highly potent pharmaceutical comprising less that 5% of the tablet (w/w), in which the highly potent pharmaceutical active is optionally selected from the group consisting of steroids, hormones, and protein and peptide therapeutics, and in which the tablet comprises a permeability enhancer.

12. A directly compressed fast dissolving tablet as claimed in any of Claims 5 to 11 in which the permeability enhancer is selected from the group consisting of bile salts such as sodium glycocholate; chitosan derivatives; or salts and derivatives of short and medium chain fatty acids (C₆-C₁₂) such as sodium caprate, which are designed to enhance the buccal and oral permeability and absorption of poorly permeable actives.

## Patentansprüche

1. Verfahren zum Herstellen einer Tablette des sich schnell auflösenden Typs, umfassend die Schritte zum Bilden eines Gemischs aus Komponenten, wobei das Gemisch mindestens 50 Gew.-% eines sich schnell auflösenden Zuckeralkohols, 1 bis 25 Gew.-% eines Superzerfallsmittels und mindestens einer aktiven Komponente umfasst, Mischen des Gemischs für eine Zeitdauer und Direktkomprimieren des gemischten Gemischs bei einer Kompressionskraft zwischen 5 und 20 kN zum Bilden einer Tablette des sich schnell auflösenden Typs, worin das gemischte Gemisch mittels biplanen Werkzeugen direkt komprimiert wird.

2. Verfahren nach Anspruch 1, worin das gemischte Gemisch zur Bildung einer Tablette des sich schnell auflösenden Typs mit einer Dicke zwischen 1 und 5 mm direkt komprimiert wird und/oder das gemischte Gemisch zur Bildung einer Tablette des sich schnell auflösenden Typs mit einem Durchmesser von zwischen 5 und 20 mm direkt komprimiert wird.

3. Verfahren nach einem der vorangehenden Ansprüche, worin der sich schnell auflösende Zuckeralkohol aus der Gruppe ausgewählt ist, umfassend: Mannitol; Sorbitol; Erythritol; Xylitol; Lactose; Dextrose; und Saccharose, und/oder der sich schnell auflösende Zuckeralkohol mindestens 60 Gew.-% oder 80 Gew.-% der Tablette ausmacht, und/oder die Tabletten zum Bilden einer Tablette des sich schnell auflösenden Typs mit einem facettierten Rand direkt komprimiert werden, und/oder eine oder mehr der Komponenten in der Form von Mikropartikeln mit einem durchschnittlichen Durchmesser von weniger als 50 µm bereitgestellt wird/werden.

4. Verfahren nach einem der vorangehenden Ansprüche, worin der Wirkstoff ein hochwirksames Pharmazeutikum ist, das weniger als 5 Gew.-% der Tablette ausmacht, und worin der hochwirksame pharmazeutische Wirkstoff gegebenenfalls aus der Gruppe ausgewählt ist, bestehend aus: Steroiden; Hormonen; Peptid-Therapeutika; und Protein-Therapeutika.

5. Sich schnell auflösende Tablette, die durch das Verfahren nach einem der Ansprüche 1 bis 4 erhältlich ist.

6. Direkt komprimierte, sich schnell auflösende Tablette, umfassend mindestens 50 Gew.-% eines sich schnell auflösenden Zuckeralkohols, 1 bis 25 Gew.-% eines Superzerfallsmittels, mindestens eine aktive Komponente und gegebenenfalls 0,1 bis 5 Gew.-% eines Schmiermittels, wobei die Tablette eine Härte von größer als 30 Newton, eine Bröckligkeit von weniger als 1 % und eine Zerfallszeit von weniger als 60 Sekunden aufweist, und wobei die Tablette flach ist.

7. Direkt komprimierte, sich schnell auflösende Tablette nach Anspruch 6 und im Wesentlichen bestehend aus:
- 50 Gew.-% bis 80 Gew.-% eines sich schnell auflösenden Zuckeralkohols;
- 2 Gew.-% bis 10 Gew.-% Superzerfallsmittel;
- 0,1 Gew.-% bis 25 Gew.-% aktiver Komponente;
- 0 Gew.-% bis 1 Gew.-% Schmiermittel, und
- gegebenenfalls einem oder mehr Geschmacksmittel(n), Fließförderungsmittel(n) oder Permeabilitätsverbesserer(n),
wobei die Tablette eine Härte von größer als 40 Newton aufweist.

8. Direkt komprimierte, sich schnell auflösende Tablette nach einem der Ansprüche 5 bis 7, wobei die Tablette einen Durchmesser zwischen 5 und 20 mm und gegebenenfalls eine Dicke zwischen 1 und 5 mm aufweist.

9. Direkt komprimierte, sich schnell auflösende Tablette nach einem der Ansprüche 5 bis 8, worin der sich schnell auflösende Zuckeralkohol aus der Gruppe ausgewählt ist, umfassend: Mannitol; Sorbitol; Erythritol; Xylitol; Lactose; Dextrose; und Saccharose, und/oder der sich schnell auflösende Zuckeralkohol mindestens 60 Gew.-% oder 80 Gew.-% der Tablette ausmacht, und/oder die Tablette einen facettierten Rand aufweist.

10. Direkt komprimierte, sich schnell auflösende Tablette nach einem der Ansprüche 5 bis 9, worin eine oder mehr der Komponenten in der Form von Mikropartikeln mit einem durchschnittlichen Durchmesser von weniger als 50 µm bereitgestellt wird/werden.

11. Direkt komprimierte, sich schnell auflösende Tablette nach einem der Ansprüche 5 bis 10. worin der Wirkstoff ein hochwirksames Pharmazeutikum ist, das weniger als 5 Gew.-% der Tablette ausmacht, worin der hochwirksame pharmazeutische Wirkstoff gegebenenfalls aus der Gruppe ausgewählt ist, bestehend aus Steroiden, Hormonen, und Protein- und Peptid-Therapeutika, und wobei die Tablette einen Permeabilitätsverbesserer umfasst.

12. Direkt komprimierte, sich schnell auflösende Tablette nach einem der Ansprüche 5 bis 11, worin der Permeabilitätsverbesserer aus der Gruppe ausgewählt ist, bestehend aus Gallensalzen, wie zum Beispiel Natriumglykocholat; Chitosanderivate; oder Salze und Derivate von kurz- und mittelkettigen Fettsäuren (C₆-C₁₂), wie zum Beispiel Natriumcaprat, die zur Verbesserung der bukkalen und oralen Permeabilität und Absorption von schlecht permeablen Wirkstoffen bestimmt sind.

## Revendications

1. Méthode de production d'un comprimé de type solubilisation rapide, comprenant les étapes consistant à former un mélange de composants, le mélange comprenant au moins 50% d'un alcool de sucre à solubilisation rapide (p/p), de 1 à 25% d'un agent super-délitant (p/p), et au moins un composant actif, mélanger le mélange pendant une période de temps, et comprimer directement le mélange malaxé à une force de compression comprise entre 5 et 20 kN afin de former un comprimé de type solubilisation rapide, dans laquelle le mélange malaxé est comprimé directement à l'aide d'outils à face plate.

2. Méthode selon la revendication 1, dans laquelle le mélange malaxé est comprimé directement afin de former un comprimé de type solubilisation rapide ayant une épaisseur comprise entre 1 et 5 mm, et/ou le mélange malaxé est comprimé directement afin de former un comprimé de type solubilisation rapide ayant un diamètre compris entre 5 et 20 mm.

3. Méthode selon l'une quelconque des revendications précédentes, dans laquelle l'alcool de sucre à solubilisation rapide est choisi dans le groupe constitué par : le mannitol ; le sorbitol ; l'érythritol ; le xylitol ; le lactose ; le dextrose ; et le saccharose ; et/ou l'alcool de sucre à solubilisation rapide constitue au moins 60% ou 80% du comprimé (p/p), et/ou les comprimés sont comprimés directement afin de former un comprimé de type solubilisation rapide ayant un bord biseauté, et/ou un ou plusieurs parmi les composants sont fournis sous forme de microparticules ayant un diamètre moyen inférieur à 50 µm.

4. Méthode selon l'une quelconque des revendications précédentes, dans laquelle le principe actif est une substance pharmaceutique hautement puissante constituant moins de 5% du comprimé (p/p), et dans laquelle la substance pharmaceutique hautement puissante est éventuellement choisie dans le groupe constitué par : les stéroïdes ; les hormones ; les peptides thérapeutiques ; et les protéines thérapeutiques.

5. Comprimé à solubilisation rapide pouvant être obtenu par la méthode selon l'une quelconque des revendications 1 à 4.

6. Comprimé à solubilisation rapide directement comprimé, comprenant au moins 50% d'un alcool de sucre à solubilisation rapide (p/p), de 1 à 25% d'un agent super-délitant (p/p), au moins un composant actif, et éventuellement de 0,1 à 5% d'un lubrifiant (p/p), où le comprimé possède une dureté supérieure à 30 Newtons, une friabilité inférieure à 1%, et un temps de désintégration inférieur à 60 secondes, et où le comprimé est à face plate.

7. Comprimé à solubilisation rapide directement comprimé selon la revendication 6, constitué sensiblement par :
- de 50% à 80% d'un alcool de sucre à solubilisation rapide (p/p) ;
- de 2 à 10% d'un agent super-délitant (p/p) ;
- de 0,1% à 25% d'un composant actif (p/p) ;
- de 0% à 1 % d'un lubrifiant (p/p) ; et
- éventuellement, un ou plusieurs agents aromatisants, améliorateurs d'écoulement ou améliorateurs de perméabilité,
le comprimé ayant une dureté supérieure à 40 Newtons.

8. Comprimé à solubilisation rapide directement comprimé selon l'une quelconque des revendications 5 à 7, où le comprimé possède un diamètre compris entre 5 et 20 mm et, éventuellement, une épaisseur comprise entre 1 et 5 mm.

9. Comprimé à solubilisation rapide directement comprimé selon l'une quelconque des revendications 5 à 8, dans lequel l'alcool de sucre à solubilisation rapide est choisi dans le groupe constitué par : le mannitol ; le sorbitol ; l'érythritol ; le xylitol ; le lactose ; le dextrose ; et le saccharose ; et/ou l'alcool de sucre à solubilisation rapide constitue au moins 60% ou 80% du comprimé (p/p), et/ou le comprimé a un bord biseauté.

10. Comprimé à solubilisation rapide directement comprimé selon l'une quelconque des revendications 5 à 9. dans lequel un ou plusieurs parmi les composants sont fournis sous forme de microparticules ayant un diamètre moyen inférieur à 50 µm.

11. Comprimé à solubilisation rapide directement comprimé selon l'une quelconque des revendications 5 à 10, dans lequel le principe actif est une substance pharmaceutique hautement puissante constituant moins de 5% du comprimé (p/p), dans laquelle la substance pharmaceutique hautement puissante est éventuellement choisie dans le groupe constitué par : les stéroïdes ; les hormones ; et les peptides et les protéines thérapeutiques, et où le comprimé comprend un améliorateur de perméabilité.

12. Comprimé à solubilisation rapide directement comprimé selon l'une quelconque des revendications 5 à 11, dans lequel l'améliorateur de perméabilité est choisi dans le groupe constitué par les sels biliaires tels que le glycocholate de sodium ; les dérivés de chitosane ; ou les sels et dérivés d'acides gras à chaîne courte et moyenne (C₆-C₁₂) tels que le caprate de sodium, qui sont conçus pour améliorer la perméabilité buccale et orale et l'absorption de principes actifs faiblement perméables.
